Europäisches Patentamt

European Patent Office

Office européen des brevets

(11) Veröffentlichungsnummer: **0 139 273**

**A2**

(12) # EUROPÄISCHE PATENTANMELDUNG

(21) Anmeldenummer: **84112023.1**

(22) Anmeldetag: **08.10.84**

(51) Int. Cl.⁴: **C 01 B 7/01**
**C 07 C 17/15**

(30) Priorität: **10.10.83 DE 3336816**

(43) Veröffentlichungstag der Anmeldung:
**02.05.85 Patentblatt 85/18**

(84) Benannte Vertragsstaaten:
**BE DE FR GB IT NL SE**

(71) Anmelder: **WACKER-CHEMIE GMBH**
**Prinzregentenstrasse 22**
**D-8000 München 22(DE)**

(72) Erfinder: **Schmidhammer, Ludwig, Dr. Dipl.-Chem.**
**Pappelweg 5**
**D-8261 Haiming(DE)**

(72) Erfinder: **Dummer, Gerhard, Dipl.-Ing.**
**Lohnerstrasse 12**
**D-8261 Burgkirchen(DE)**

(72) Erfinder: **Haselwarter, Klaus, Dipl.-Ing.**
**Marktlerstrasse 7**
**D-8263 Burghausen(DE)**

(72) Erfinder: **Strasser, Rudolf, Dr. Dipl.-Chem.**
**Lindacherstrasse 58**
**D-8263 Burghausen(DE)**

(54) **Verfahren zum Aufbereiten von chlor-, ehtylen- und/oder acetylenhaltigem Chlorwasserstoff.**

(57) Die Erfindung betrifft ein Verfahren zur Aufbereitung von Chlorwasserstoff, der Chlor, Eisen-III-Chlorid, Acetylen und/oder Ethylen enthält, für einen Oxichlorierungsprozeß, wobei bei Temperaturen von 120 bis 120 bis 180°C innerhalb eines Zeitraumes von maximal 0,9 Sekunden, nachdem sowohl Acetylen und/oder Ethylen gleichzeitig mit Chlor im Chlorwasserstoff vorliegt, gasförmiges Wasser zugemischt wird.

Croydon Printing Company Ltd.

0139273

Wacker-Chemie GmbH    München, den 07.09.1983
PAT/Dr.Ra/hu
Wa 8233-C


Verfahren zum Aufbereiten von chlor-, ethylen- und/oder
acetylen-haltigem Chlorwasserstoff.

---

Die Erfindung betrifft ein Verfahren zur Aufbereitung von
Chlor, Eisen-III-Chlorid, sowie ethylen-und/oder acetylen-
haltigem Chlorwasserstoff.


Chlorwasserstoff wird in großen Mengen zur Herstellung von
1.2-Dichlorethan durch Oxichlorierung von Ethylen verbraucht.
Dieser Chlorwasserstoff kann aus mehrerlei Quellen
stammen und enthält je nach Herkunft unterschiedliche Verunreinigungen: Chlorwasserstoff, der durch thermische Spaltung von Dichlorethan zu Vinylchlorid gewonnen wurde, enthält stets kleinere Mengen an Acetylen oder dessen Hydrierungsprodukt Ethylen. Chlorwasserstoff, der aus substituierenden Chlorierungsreaktionen stammt, beispielsweise aus
der Chlorierung von Essigsäure oder Aromaten, oder aus
Perchlorierungsprozessen von $C_1$ bis $C_3$ Kohlenwasserstoffen
enthält stets elementares Chlor. Das gleiche gilt für
Chlorwasserstoff, der bei der Verbrennung von Chlorkohlenwasserstoffen, z. B. bei der Verbrennung von Chlorkohlenwasserstoff-Destillationsrückständen, anfällt.


Nachdem der Chlorwasserstoff üblicherweise in stählernen
Leitungen geführt wird, finden sich in mit Chlor verunreinigtem Chlorwasserstoff stets zumindest geringe Mengen
an Eisenchlorid, die durch den korrosiven Angriff des
Chlors auf Stahl gebildet werden. Dieser Effekt tritt im Dauerbetrieb einer Anlage auch dann auf, wenn der Chlorwasserstoff vorher einer konventionellen Chlorreinigung, beispielsweise einer Wäsche in Tetrachlorkohlenstoff oder einer adsorptiven Reinigung an Aktivkohlefiltern, unterworfen wurde. Bei derartigen Reinigungsschritten schlagen nämlich immer noch geringe Mengen, etwa Mengen in zweistelligem ppm-Bereich, an Chlor durch.

Wird nun chlor- und damit eisen-III-chlorid-haltiger Chlorwasserstoff (zumindest, wenn wie üblich, stählerne Leitungen benutzt werden) in die Vorwärmstufe für eine Oxichlorierung gebracht und mit Acetylen oder Ethylen, die aus beliebigen Quellen stammen können, vermischt, so kommt es innerhalb kurzer Betriebszeiten zur Ablagerung von ruß- oder koksähnlichen Zersetzungsprodukten an Leitungen, meßtechnischen Einrichtungen und letztlich auch im Katalysatorbett des Oxichlorierungskatalysators.

Die Folgen sind hinlänglich bekannt: Es kommt zu Betriebsstörungen, unter Umständen explosionsgefährdeten Betriebszuständen aufgrund von Fehlmessungen, Desaktivierung des Oxichlorierungskatalysators, Druckanstieg im Katalysatorbett u. dgl.

Aufgabe der Erfindung war es, die oben dargestellten Verrußungs- oder Verkokungserscheinungen zu verhindern.

Es wurde nun gefunden, daß durch Zusatz von gasförmigem Wasser zu chlor-, eisen-III-chlorid-, sowie ethylen- bzw. acetylen-haltigem Chlorwasserstoff die genannte Aufgabe gelöst wird.

Gegenstand der Erfindung ist ein Verfahren zur Aufbereitung von chlor-, eisen-III-chlorid-, sowie acetylen- und/oder
ethylen-haltigem Chlorwasserstoff, das dadurch gekennzeichnet ist, daß bei Temperaturen von 120 bis 180°C innerhalb eines Zeitraumes von maximal 0,9 Sekunden nachdem
sowohl Acetylen und/oder Ethylen als auch Chlor und Eisen-
III-Chlorid im Chlorwasserstoff vorliegen, gasförmiges
Wasser in Mengen von 5 bis 20 Mol-%, bezogen auf die Gesamtmenge des Chlorwasserstoffs zugemischt werden.

Der erfindungsgemäß zu behandelnde Chlorwasserstoff enthält
Chlormengen von 20 Vol.-ppm bis 200 Vol.-ppm, insbesondere 30 Vol.-ppm bis 80 Vol.-ppm, jeweils bezogen auf das
Gesamtvolumen des Chlorwasserstoffs und Mengen an Eisen-
III-Chlorid von 1 bis 4 mg pro Nm³. Sofern die oben
angegebenen Mengen für Chlor und Eisen-III-Chlorid überschritten werden, wird der Chlorwasserstoff vorher einer
konventionellen Reinigung unterzogen, beispielsweise durch
Überleiten des Chlorwasserstoffs über Aktivkohlefilter,
durch Wäsche des Chlorwasserstoffs in Tetrachlorkohlenstoff,
durch Reinigen des Chlorwasserstoffs in azeotrop destillierender Salzsäure und dergleichen.

Die Mengen an Acetylen sind in der Regel nicht höher, als die
beim Crackprozeß von 1.2-Dichlorethan als Nebenprodukt anfallenden Mengen, also etwa 0,05 bis 0,5 Vol.-%, sofern
Chlorwasserstoff aus solchen Quellen zugemischt wird.

Die Mengen an Ethylen bewegen sich in weiten Grenzen und
reichen von den oben für Acetylen genannten Beträgen bis
in etwa für die Oxichlorierung der Gesamtmenge vorliegenden Chlorwasserstoffs benötigten Ethylenmengen.

Es werden erfindungsgemäß Temperaturen von 120 bis 180°C eingehalten, die sowohl für die Vorwärmung des Reaktionsgemisches für die Oxichlorierung erforderlich sind, als
auch um Taupunktsunterschreitungen zu verhindern.

Die Menge eingesetzten, dampfförmigen Wassers beträgt 5 bis
20 Mol-%, bezogen auf die Gesamtmenge der einer Oxichlorierung
zuzuführenden Chlorwasserstoffs. Es wird ausdrücklich
darauf hingewiesen, daß Wasser im erfindungsgemäßen Reaktionssystem ausschließlich im gasförmigen Zustand vorliegt.

Die Wasserdampfzugabe erfolgt spätestens 0,9 Sekunden, insbesondere 0,3 bis 0,8 Sekunden nachdem sowohl Chlor und damit
Eisen-III-Chlorid als auch die oben genannten Mengen an
Acetylen und/oder Ethylen im Chlorwasserstoff vorliegen und
das Reaktionsgemisch eine Temperatur von mindestens 120°C
aufweist.

Nachdem die erfindungsgemäßen Maßnahmen der Aufbereitung des
Chlorwasserstoffs für eine Oxichlorierung dienen, wird das
gasförmige Wasser zweckmäßigerweise zusammen mit der für
den Oxichlorierungsprozeß erforderlichen Menge an Sauerstoff
bzw. mit dem entsprechenden Luftstrom in das System eingespeist. Alternativ kann die Wasserdampfzufuhr auch über den
ethylen- oder den ethylen- und/oder acetylen-haltigen Gasstrom erfolgen. Selbstverständlich kann auch eine seperate
Wasserdampfzugabe erfolgen, solange die erfindungsgemäßen
Verweilzeiten von maximal 0,9 Sekunden eingehalten werden.

Das derart aufbereitete Gemisch wird schließlich in das
Oxichlorierungssystem eingeleitet.

Nach dem erfindungsgemäßen Verfahren gelingt es, die Zuleitungen zum Oxichlorierungssystem und ebenso den Oxichlorierungskatalysator selbst frei von Ruß- und Koksablagerungen zu halten.

Die Erfindung wird nun anhand von Beispielen näher erläutert.

## Beispiel 1

100 kmol/h Chlorwasserstoff aus einer Perchlorierungsanlage mit einem Gehalt von 30 Vol. ppm Chlor und 5 mg $FeCl_3$ pro $Nm^3$ wurden bei 20°C über ein Aktivkohlebett geleitet. Danach wurde der Chlorwasserstoff über einen Wärmetauscher aus Stahl geleitet und auf 170°C vorgewärmt. Am Austritt des Wärmetauschers enthielt der Chlorwasserstoff weiterhin 30 Vol. ppm Chlor und 1,5 mg $FeCl_3$ pro $Nm^3$. Danach wurde der vorgewärmte Chlorwasserstoff mit 55 kmol/h auf 40°C vorgewärmten Ethylens abgemischt. Unmittelbar danach erfolgte die Zugabe eines Gemisches von 54 kmol/h Luft und 13 kmol/h Wasserdampf, das auf 180°C vorgewärmt war. Die Verweilzeit des Reaktionsgemisches zwischen Ethylenzugabe und Luft/Wasserdampfzugabe betrug 0,3 Sekunden. Das derart aufbereitete Dampfgemisch strömte schließlich einer aus drei Reaktoren bestehenden Festbett-Oxichlorierungsanlage zu, die aus drei in Reihe geschalteten Reaktoren bestand. Die insgesamt erforderlichen weiteren 80 kmol/h Luft für eine quantitative Umsetzung von Chlorwasserstoff mit Ethylen zu 1.2-Dichlorethan erfolgte zwischen dem 1. und 2. und 2. und 3. Reaktor.

Die Aktivität des Katalysators war auch nach einem Jahr Laufzeit noch nicht beeinträchtigt. Es konnten keinerlei ruß- oder koksartige Ablagerungen am Eintritt des Reaktors oder im Reaktor festgestellt werden.

## Vergleichsbeispiel 1

Die Arbeitsweise gemäß Beispiel 1 wurde wiederholt, mit der Abänderung, daß im Reaktionssystem kein Wasserdampf zugemischt wurde.

Nach zweimonatiger Standzeit wurde bereits eine Katalysatorverdichtung im 1. Reaktor durch ansteigenden Druckverlust
festgestellt. Gleichzeitig wurde die unerwünschte Nebenproduktbildung von Ethylchlorid und Vinylchlorid, sowie
ein Absinken des Chlorwasserstoff-Umsetzungsgrades im
Oxichlorierungsprozeß festgestellt. Nach 6 monatiger
Laufzeit mußte die Anlage wegen zu hoher Druckverluste im
1. Reaktor abgestellt werden.

Vergleichsbeispiel 2

Die Arbeitsweise gemäß Beispiel 1 wurde wiederholt, mit der
Abänderung, daß die Verweilzeit des bereits mit Ethylen
abgemischten Chlorwasserstoffs bis zur Zugabe des Luft/
Wasserdampfgemisches eine Sekunde betrug.

Nach einer Laufzeit von 9 Monaten mußte der Katalysator im
1. Reaktor gewechselt werden, nachdem sich koksartige Ablagerungen in einer Menge gebildet hatten, die einen nicht
mehr tragbaren Anstieg des Druckverlustes im 1. Reaktor zur
Folge hatten.

Beispiel 2

110 kmol/h Chlorwasserstoff aus einer Perchlorierungsanlage
wurden über ein Aktivkohlebett geleitet. Der Chlorgehalt
betrug danach 50 Vol. ppm, der $FeCl_3$-Gehalt betrug 1,2 mg
pro $Nm^3$. Der Chlorwasserstoff wurde auf 155°C vorgewärmt
und mit 300 kmol/h eines Chlorwasserstoffs abgemischt, der
aus einer 1.2-Dichlorethan-Crackanlage stammte und 2000
Vol. ppm Ethylen, 400 Vol. ppm Ethan und 10 Vol. ppm
Acetylen enthielt. Der Crack-Chlorwasserstoff wies eine
Temperatur von 160°C auf. Das Gemisch wurde mit 235 kmol/h

0139273

Ethylen abgemischt. Danach erfolgte die Zugabe eines Gemisches aus 226 kmol/h Luft und 45 kmol/h Wasserdampf
derart, daß die Verweilzeit zwischen 1. Zugabe von Ethylen
bzw. Acetylen bis zur Wasserdampfzugabe 0,75 Sekunden betrug.
Schließlich wurde das System einem Oxichlorierungsreaktor
aufgegeben.

Nach achtzehnmonatiger Laufzeit konnten weder Koksablagerungen in den Zuleitungen der vereinigten Chlorwasserstoffströme noch im Bereich des 1. Reaktors festgestellt werden.

Vergleichsbeispiel 3

Es wurde die Arbeitsweise gemäß Beispiel 2 wiederholt, mit
der Abänderung, daß die Verweilzeit zwischen 1. Ethylenzugabe zum aus einer Perchlorierung stammenden Chlorwasserstoff bis zur Wasserdampfzugabe 1,5 Sekunden betrug.

Nach einer Laufzeit von einem Monat wurden bereits Koksablagerungen an einer Meßblende festgestellt, die sich im
Leitungssystem der vereinigten Chlorwasserstoffströme befand.

C139273

Patentansprüche

1. Verfahren zur Aufbereitung von chlor-, eisen-III-chlorid-,
   sowie acetylen- und/oder ethylen-haltigem Chlorwasserstoff, d a d u r c h   g e k e n n z e i c h n e t ,
   daß bei Temperaturen von 120 bis 180°C innerhalb eines
   Zeitraumes von maximal 0,9 Sekunden,nachdem sowohl
   Acetylen als auch Ethylen gleichzeitig mit Chlor im
   Chlorwasserstoff vorliegen, gasförmiges Wasser in Mengen
   von 5 bis 20 Mol.-%, bezogen auf die Gesamtmenge des
   Chlorwasserstoffs, zugemischt werden.

2. Verfahren nach Anspruch 1, d a d u r c h   g e k e n n -
   z e i c h n e t ,   daß die Verweilzeit 0,3 bis 0,8 Sekunden beträgt.